(19) Europäisches Patentamt
European Patent Office
Office européen des brevets

(11) Numéro de publication: **0 087 378**
**B1**

(12) **FASCICULE DE BREVET EUROPEEN**

(45) Date de publication du fascicule du brevet: **26.10.88**

(51) Int. Cl.$^4$: **A 61 K 31/13,** A 61 K 31/15, C 07 C 119/06, C 07 C 93/02

(21) Numéro de dépôt: **83440013.7**

(22) Date de dépôt: **11.02.83**

# (54) Ethers-oximes d'alcoylaminoalcools comme médicaments, produits nouveaux et procédés pour leur préparation.

(30) Priorité: **19.02.82 FR 8202740**

(43) Date de publication de la demande:
**31.08.83 Bulletin 83/35**

(45) Mention de la délivrance du brevet:
**26.10.88 Bulletin 88/43**

(84) Etats contractants désignés:
**AT BE CH DE FR GB IT LI NL SE**

(56) Documents cités:
**EP-A-0 037 777**
**GB-A-1 531 420**

**JOURNAL OF MEDICINAL CHEMISTRY, vol. 23, no. 6, juin 1980, pages 620-624, American Chemical Society, Washington, D.C., US, G. LECLERC et al.: "Synthesis and beta-adrenergic blocking activity of new aliphatic oxime esthers"**

**Meth. and Find Exptl. Clin. Pharm. 1985, 7(4), pp. 195-201**

**Le dossier contient des informations techniques présentées postérieurement au dépôt de la demande et ne figurant pas dans le présent fascicule.**

(73) Titulaire: **Laboratoires P.O.S. Société Anonyme dite:**
**21 rte de Lapoutroie**
**F-68240 Kaysersberg (FR)**

(72) Inventeur: **Leclerc, Gérard**
**5 rue Louis Appfel**
**F-67000 Strasbourg (FR)**
Inventeur: **Bouzoubaa, Mohammed**
**13 boulevard de la Marne**
**F-67000 Strasbourg (FR)**
Inventeur: **Andermann, Guy**
**5 rue des Jonquilles**
**F-68000 Colmar (FR)**
Inventeur: **de Burlet, Georges**
**7 rue Christian Pfister**
**F-68980 Beblenheim (FR)**
Inventeur: **Cannet, Catherine**
**4 rue Saint Léon**
**F-68000 Colmar (FR)**
Inventeur: **Himber, Jacques**
**101 rue Théodore Deck**
**F-68500 Guebwiller (FR)**

(74) Mandataire: **Cuer, André**
**CABINET CUER 30, rue de Léningrad**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**EP 0 087 378 B1**

Courier Press, Leamington Spa, England.

**Description**

La présente invention a trait au domaine des médicaments. Elle concerne des éthers-oximes d'alcoyl-aminoalcools comme ingrédients actifs de compositions médicamenteuses destinées notamment au traitement des maladies de l'oeil dont en particulier la glaucome.

On a déjà signalé l'activité béta-bloquante et les propriétés antihypertensives d'ortho-amino-oximes aromatiques de formule générale: A = N—O—$CH_2$—CHOH—$CH_2$—NH—R, dans laquelle R est un reste aliphatique alors que A est un radical renfermant au moins un noyau aromatique (brevet français FR—A—2300555). Toutefois, lorsqu'on utilise de tels dérivés ou leurs sels pharmaceutiquement acceptables dans le traitement des glaucomes, comme par exemple le maléate de timolol, on constate souvent des effets secondaires indésirables tels que, notamment, des troubles bronchiques (asthme) ou cardiovasculaires (hypotension orthostatique, ralentissement du pouls).

On connait par ailleurs, en tant que produits chimiques, des ortho-amino-oximes dans lesquelles le reste A ci-dessus peut-être aliphatique ou alicyclique en $C_6$ (G. Leclerc & al. J. Med. chem 23, 6, p. 620—624, 1980). Cependant, les essais "in vitro", effectués avec certains de ces produits, sur des tissus d'animaux, pour évaluer l'activité béta-bloquante, ne sont pas significatifs pour l'application anti-glaucome.

On connait enfin des alcoxypropanolamines de formule $R_1$—O—$CH_2$—CHOH—$CH_2$—NH—R dans lesquelles $R_1$ est un radical alcoyle, alcényle ou alcynyle. Ces composés sont présentés dans la demande de brevet européen EP—A—0037780 comme ayant une activité béta-bloquante utile dans le traitement des troubles cardio-vasculaires, mais ils ne peuvent être utilisés en application thérapeutique sur l'oeil, du fait de leur mauvaise tolérance locale et leur pouvoir anesthésique, ce qui est un grand inconvénient pour un collyre visant à traiter le glaucome.

Or, il a maintenant été trouvé que certaines amino-oximes aliphatiques présentaient de remarquables propriétés antihypertensives et antiglaucomateuses, sans conséquences secondaires dans l'emploi comme cela est le cas pour des produits homologues.

L'invention a donc pour objet, à titre de médicaments pour le traitement des maladies de l'oeil, certains éthers-oximes d'alcoylaminoalcools, ainsi que leurs sels pharmaceutiquement acceptables, appartenant à la formule:

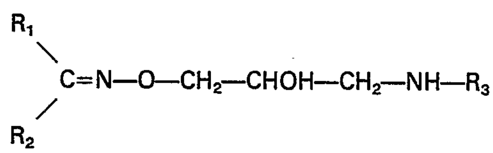 (1)

dans laquelle:

— $R_1$ et $R_2$ peuvent être:

a) soit tous deux des restes cycloalcoyles $C_3$ à $C_5$;

b) soit l'un, un reste alcoyle $C_1$ à $C_5$ et l'autre un reste cycloalcoyle $C_3$ à $C_5$;

c) $R_1$ et $R_2$ peuvent former ensemble un cyclohexane ou un 3,3,5-triméthylcyclohexane.

— $R_3$ est un radical alcoyle $C_1$ à $C_5$ linéaire ou ramifié.

L'invention a trait également à certains produits industriels nouveaux appartenant à la formule (1).

En pratique, on peut citer à titre non limitatif, comme produits entrant dans le cadre précité, des composés pour lesquels: $R_3$ est un radical éthyle, propyle ou, de préférence, isopropyle et tertiobutyle; $R_1$ = $R_2$ = cyclopropyle; $R_1$ = $R_2$ = cyclopentyle; $R_1$ = cyclopropyle alors que $R_2$ est un radical méthyle ou éthyle ou propyle ou butyle; $R_1$ = cyclopropyle substitué par un alcoyle inférieur et $R_2$ est un radical alcoyle du même type que ci-dessus.

L'invention a également pour objet un procédé pour la préparation des composés de formule (I).

Selon ce procédé on fait réagir la cétone de formule

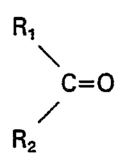

avec l'hydroxylamine pour obtenir le composé de formule (II):

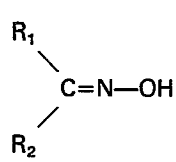

qui par réaction avec une épihalohydrine conduit au composé de formule (III):

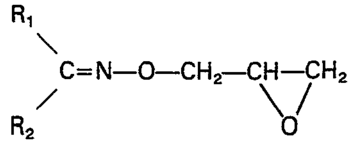

(III)

qui par réaction avec une amine $R_3$—$NH_2$ conduit au composé (I), $R_1$, $R_2$ et $R_3$ ayant les mêmes significations que précédemment.

PARTIE EXPERIMENTALE

Les composés suivants ont été synthétisés, en particulier:

— N-tert-butyl amino-1, cyclopropyl methylcétone oximino-3, propanol-2 (sous forme d'oxalate) composé N° 1

— N-tert-butyl amino-1, dicyclopropyl cétone oximino-3, propanol-2, (sous forme de fumarate), composé N° 2

— N-tert-butyl amino-1, ol-2,. (3,3,5-trimethyl, 1-oximino cyclohexane)-3, propane (sous forme d'oxalate), composé N° 3.

On indique dans le tableau 1 ci-après les caractéristiques essentielles de ces composés.

On a synthétisé le composé N° 1 sous ses deux formes isomères optiques S(−) et R(+) avec les caractéristiques suivantes:

S(−): — pouvoir rotatoire: $\alpha_D^{20°} = -3,3°$
— PF = 133°C

R(+): — pouvoir rotatoire: $\alpha_D^{20°} = +2,7°$
— PF = 130°C (environ)

L'invention concerne également les énantiomères stéréospécifiques syn et anti.

TABLEAU N° 1

| Composé N° | Base | Acide | point de fusion F °C | Calculé (%) C | H | N | Trouvé (%) C | H | N | Rendement % | Solvant de recristallisation |
|---|---|---|---|---|---|---|---|---|---|---|---|
| 1 | 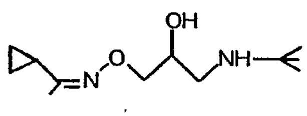 | 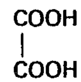 | hemioxalate 174 | 57,12 | 9,12 | 10,28 | 56,86 | 9,35 | 10,2 | 27 | AcOEt/MeOH |
| | | | oxalate 128 | 51,81 | 8,23 | 8,80 | 52,63 | 8,11 | 8,63 | 27 | AcOEt/MeOH |
| 2 | 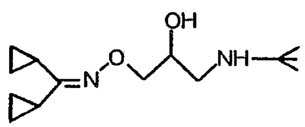 | 1/2 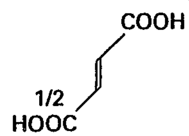 | 159 | 61,56 | 9,04 | 9,00 | 61,47 | 9,14 | 9,00 | 15 | AcOEt |
| 3 | 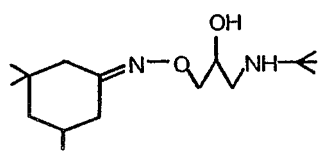 | 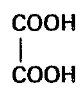 | 88 | 57,73 | 9,15 | 7,48 | 57,65 | 9,17 | n.c. | 20 | Benzène/Oxyde d'isopropyle |

Quelques exemple de préparation sont donnés ci-après:

I. Préparation de l'oxalate du N-tert-butyl amino-1, cyclopropyl methylcétone oximino-3, propanol-2 (composé N° 1)

L'oxime est préparée à partir de la cyclopropylmethyl-cétone 4,5 g (0,05 mol), le chlorhydrate d'hydroxylamine 7 g, (0,05 × 2 mol) et 8,2 g (0,05 × 2) d'acétate de sodium qui sont préalablement dissous dans 30 cc d'eau distillée; le mélange est agité à 40°C pendant 12 heures, puis on extrait deux fois à l'AcOEt, on évapore le solvant sous le vide de la trompe à eau (faible chauffage 30°C), on reprend avec de l'eau (minimum d'eau), on neutralise l'acide acétique formé dans la réaction avec du bicarbonate de potassium, on extrait deux fois à l'acétate d'étyle, on sèche sur $MgSO_4$, puis filtre et évapore l'AcOEt, l'oxime obtenu est pure, rendement 88% (4,7 g d'oxime brut). On dissout l'oxime dans 100 ml de THF anhydre, on ajoute par petites quantités du NaH (0,05 mol). On ajoute 3,9 ml d'épibromhydrine en solution dans 10 ml de THF anhydre sur la solution du sel d'oxime préalablement refroidi dans un bain de glace; après addition à froid, on laisse agiter pendant 48 heures à température ambiante, on filtre le bromure de sodium formé, on évapore le THF sous le vide de la pompe à eau; ensuite, l'époxyde brut est mis en solution dans de l'EtOH absolu, on ajoute (5,2 × 2 ml) de tert-butylamine, on laisse agiter à température ambiante pendant 24 heures, on évapore l'éthanol; la base brute est ensuite dissoute dans un minimum d'une solution d'acide chlorhydrique 1N, on extrait deux fois à l'éther les impuretés neutres et acides, on neutralise la phase aqueuse avec de $K_2CO_3$, on sature cette même solution avec $K_2CO_3$, on extrait trois fois à l'AcOEt, puis on sèche sur $MgSO_4$, on filtre et évapore l'AcOEt. La base brute est mise en solution dans un minimum d'AcOEt/ether (95/5), on ajoute 6,30 g d'acide oxalique dissous dans un minimum d'AcOEt, on filtre l'oxalate formé, on recristallise dans un mélange AcOEt/MeOH (80/20). 4,3 g d'oxalate sont récupérés après séchage "à la pompe", pression réduite 0,013 mbar (0,01 mm Hg), pendant deux heures. Rendement total de la réaction: 27%.

On a préparé de la même manière le composé N° 2.

II. Préparation de l'oxalate du N-tert-butyl amino-1, (3,3,5-trimethyl, 1 oximino cyclohexane)-3 propanol-2 (composé N° 3)

L'oxime est préparée à partir de la triméthyl-3,3,5 cyclohexanone 10 g (0,07 mol), le chlorhydrate d'hydroxylamine (0,07 × 2 mol), acétate de sodium (0,07 × 2 mol) qui sont préalablement dissous dans un minimum d'eau distillée. Le mélange est agité pendant 2 heures à 40°C, puis on refroidit le mélange, et filtre l'oxime, on lave avec 3 × 5 ml d'eau. On fait redissoudre l'oxime dans de l'acétate d'éthyle, et l'on sèche sur $MgSO_4$, filtre et évapore à sec. Rendement 90%. On fait alors dissoudre l'oxime obtenu dans 100 à 200 ml de THF anhydre, on ajoute par petites quantités du NaH (0,063 mol) pour former le sel de sodium. On ajoute goutte à goutte une solution d'epibromhydrine dans THF (0,063 mol d'epibromhydrine). Sur la solution du sel d'oxime préalablement refroidi dans un bain de glace, après addition, on laisse agiter 48 heures à température ambiante. On filtre le bromure de sodium formé, on évapore le THF. L'époxyde brut est mis en solution dans l'éthanol absolu, on ajoute (2 × 0,063 mol) de tertbutylamine, on laisse agiter à température ambiante pendant 24 heures, on évapore l'éthanol et la tertbutylamine en excès. La base brute est dissoute dans un minimum d'une solution d'acide chlorhydrique 1N, on extrait deux fois à l'éther les impuretés neutres et acides, on neutralise la phase aqueuse avec du $K_2CO_3$, on sature cette même solution avec du $K_2CO_3$, on extrait trois fois à l'AcOEt, on sèche sur $MgSO_4$, on filtre, on évapore le solvant. La base (0,032 mol) brute est mise en solution dans un mélange AcOEt/éther (95/5), on ajoute de l'acide oxalique dissous dans l'AcOEt (0,032 mol). On filtre l'oxalate, on recristallise dans l'AcOEt-oxyde d'isopropyle. Rendement global de la synthèse: 20%.

Des médicaments à base de produits susvisés et destinés à l'administration par voie locale, sous forme de solutions aqueuses, collyres, suspensions aqueuses et pommades ophtalmiques, se sont révélés très efficaces comme antihypertensifs et antiglaucomateux, en particulier lors du traitement des glaucomes lorsque la pression intraoculaire est supérieure à 39,2 mbar (22 mm de mercure). Des séries d'expériences et observations faites sur des collyres préparés selon l'invention avec 0,1 à 1% en poids d'argent actif, dans les domaines toxicologique, pharmacologique, thérapeutique ont donné des résultats très intéressants.

A. TOXICOLOGIE

Les expériences d'administration par voie orale et par voie intrapéritonéale à des souris et à des rats ont montré une absence totale de toxicité aigüe et une dose léthale DL 50 nettement supérieure aux doses maximales d'administration. Le test cytogénétique de mutagénèse (ou test micronucleus) s'est avéré négatif chez le rat. En outre, l'indice d'irritation oculaire est pratiquement nul chez le lapin et l'on a pu conclure à une très bonne tolérance dans l'oeil humain.

B. PHARMACOLOGIE — APPLICATIONS PHARMACEUTIQUES

Les résultats d'expérimentations sur les lapins ont été hautement significatifs. Les essais de mesure de degré d'allergénicité (test de Magnusson), effectués sur cobayes, ont montré qu'il ne pouvait se produire aucun accident allergique.

Par ailleurs, les expériences d'activité mutagène sur diverses bactéries ont permis de conclure à l'absence de pouvoir mutagène des substances conformes à l'invention.

Enfin, les nombreuses études multicentriques effectuées sur l'homme ont mis en évidence la grande efficacité des médicaments de l'invention.

En pratique, les compositions selon l'invention sont particulièrement aptes à l'utilisation comme collyres. Outre le constituant actif (ou mélange) choisi dans la classe susvisée de formule (I), le collyre peut renfermer divers autres constituants à fonctions diverses et jouant notamment les rôles: d'agents isotoniques, comme par exemple le chlorure de sodium ou le chlorure de potassium; d'agents tampons, comme par exemple les phosphates de sodium ou de potassium, le borate de sodium; éventuellement d'agents conservateurs comme par exemple des sels de mercure non toxiques, les ammoniums quaternaires, les sels de chlorhexidine. Bien entendu, comme dans le cas des autres collyres, les compositions sont diluées par de l'eau distillée.

La dose usuelle, variable selon le sujet à traiter et l'affection en cause, peut être par exemple de 1 à 4 applications par jour, d'un collyre renfermant de 0,1% à 1% de principe actif, chez l'homme.

A titre d'exemple non limitatif des propriétés et de l'application de compositions selon l'invention, on indiquera ci-dessous les résultats résumés de l'étude pharmacologique, en vue de l'activité antiglaucomateuse, entreprise sur des lapins albinos avec divers collyres selon l'invention, par comparaison avec des collyres ayant les mêmes concentrations en éthers-oxydes aromatiques d'aminoalcools de type connu, notamment le maléate de timolol, de formule ci-dessous:

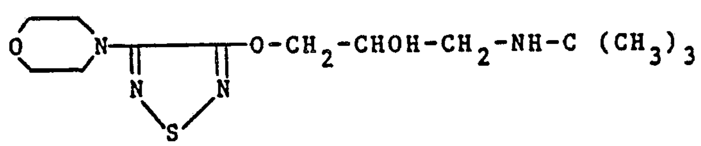

Certains collyres expérimentés selon l'invention avaient la composition suivante:

| | |
|---|---|
| agent actif | 0,1 à 1 g% |
| créatinine | 0,5 g% |
| acide citrique | 0,5 g% |
| chlorure de sodium | 0,225 g% |
| hydroxyéthylcellulose | 0,2 g% |
| soude . . . quantité pour avoir pH 7,3 eau purifiée q.s.p. pour | 100 ml |

Une autre composition utilisable est par exemple en % en poids:

| | |
|---|---|
| agent actif | 0,5 |
| $PO_4$ monopotassique | 0,113 |
| $PO_4$ dipotassique | 0,95 |
| chlorure de sodium | 0,63 |
| chlorure de benzalkonium | 0,01 |
| eau purifiée q.s.p. | 100 ml. |

Parmi les agents actifs de formule (I) on a expérimenté entre autres les produits 1 à 3 du tableau 1 précité.

Chez l'animal, les expérimentations ont été menées sur des séries de lapins albinos néozélandais de 3,5 à 4,5 kg en opérant en double aveugle avec chacune des dilutions ci-dessus et avec des repos thérapeutiques entre les traitements. Des mesures sans traitement ont également été faites, par anesthésie locale de l'oeil des lapins avec de la posicaine, afin de pouvoir suivre l'évolution de la P.I.O. (pression intra-oculaire) chez les animaux.

On a provoqué un glaucome dans les yeux droits des lapins par injection intra-oculaire de: alpha-chymotrypsine selon la méthode de SEARS (Am. J. Ophtalmol 1974, 71, p. 378) modifiée par VAREILLES & al (Am. J. Ophtalmol 1979, 2 10, p. 561).

Les P.I.O. étaient mesurées toutes les demi-heures au cours des trois premières heures puis toutes les heures jusqu'à la 7ème heure. Parallèlement, on mesurait l'effet d'anesthésie locale, chaque mesure étant faite sur les deux yeux en même temps, les résultats de l'oeil gauche non traité servant d'essais-témoins.

L'ensemble des résultats obtenus n'est pas donné ici avec tous les détails mais il peut être résumé comme suit:

— Pour pratiquement, toutes les compositions de collyre selon l'invention avec l'un quelconque des agents actifs précités, on obtient une efficacité supérieure à celle des dérivés du Timolol, à concentrations égales, dans l'activité antiglaucomateuse; ceci joint à une grande rapidité d'action. En moyenne, pour les agents actifs, la P.I.O. passait par un minimum trois heures après l'instillation et ne retrouvait pas sa valeur initiale après un temps de sept heures.

— l'effet anesthésique produit par les diverses compositions sur la cornée de lapin était généralement nul pour les composés de l'invention.

— l'action hypotensive ne variait pas de manière significative avec la concentration pour tous les produits expérimentés.

— l'efficacité des produits actifs selon l'invention passait par un maximum pour les diverses concentrations précitées, la dose optimum étant de 0,1%, 0,25% ou encore 0,50% selon les cas (% par rapport au poids total de collyre).

L'activité béta-bloquante a été mise en évidence par la mesure des $PA_2$.

La $PA_2$ est le cologarithme de la concentration d'antagoniste en présence de laquelle il faut deux fois plus d'agoniste qu'en son absence pour obtenir le même effet, voir à ce sujet: "Calcul des PAx", fiche technique N° 16 J. Pharmacol. Paris 1971, 2, 3, 373—380.

Elle a été mesurée sur l'oreillette isolée du cobaye et sur la trachée isolée du cobaye.

Les résultats sont repris dans le tableau 2 ci-après. Le produit utilisé à titre comparatif est le composé suivant:

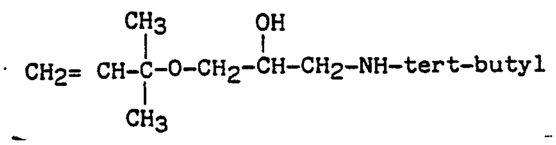

Ce composé est du type faisant l'objet de la demande de brevt européen EP—A—0037780.

<u>Tableau N° 2</u>

| Composé N° | Oreillette de cobaye | | Trachée $pA_2$ |
|---|---|---|---|
| | Chronotrope $pA_2$ | Inotrope $pA_2$ | |
| 1 | 8,7 | 9,0 | 9,04 |
| 2 | 8,3 | 8,14 | 8,67 |
| Comparatif | 4,43 | 4,32 | 4,96 |

Chez l'homme l'étude d'activité sur la pression intra-oculaire normale a montré que les médicaments testés faisaient chuter rapidement la pression intra-oculaire après instillation d'une seule goutte de collyre. Chez les malades atteints de glaucome chronique à angle ouvert, la chute tensionnelle moyenne était de 17,3 à 21,3 mbar (13 à 16 mm Hg) pour une instillation de collyre dosé à 0,5% en principe actif.

Les essais cliniques n'ont pas permis de mettre en évidence les effets secondaires tels que par exemple troubles bronchiques ou cardiovasculaires (hypotension orthostastique, ralentissement du pouls) constatés consécutivement à l'emploi d'éthers-oximes de constitution voisine de celle des produits de l'invention et plus généralement des béta-bloquants de l'art antérieur.

Les résultats des essais conduits avec le composé N° 1 sont repris dans le tableau 3 ci-après.

TABLEAU No. 3

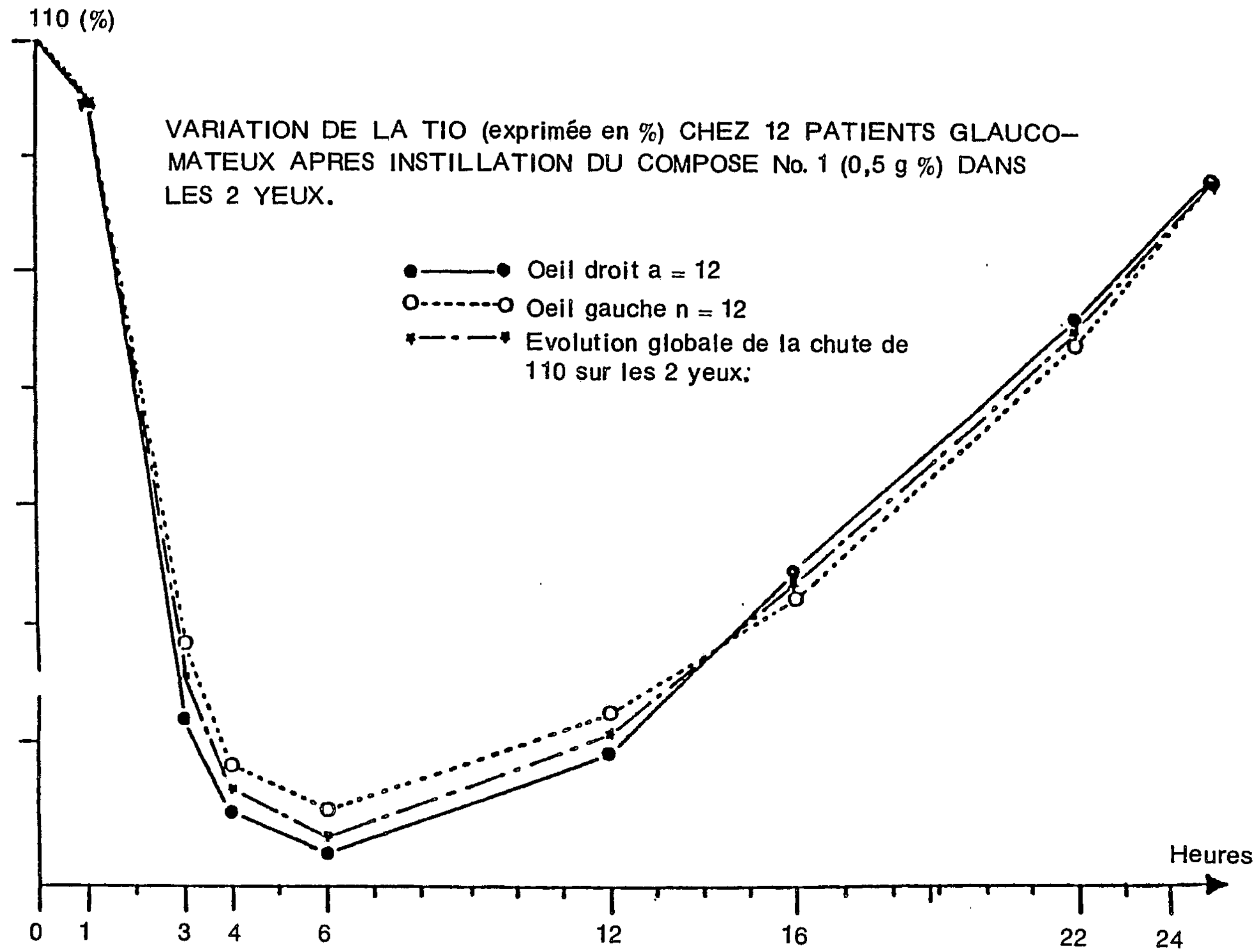
110 (%)
VARIATION DE LA TIO (exprimée en %) CHEZ 12 PATIENTS GLAUCO-MATEUX APRES INSTILLATION DU COMPOSE No. 1 (0,5 g %) DANS LES 2 YEUX.
Oeil droit a = 12
Oeil gauche n = 12
Evolution globale de la chute de 110 sur les 2 yeux;
Heures
0
1
3
4
6
12
16
22
24

**Revendications**

1. Utilisation pour la fabrication d'un médicament pour le traitement des maladies de l'oeil, dont le glaucome, d'éthers-oximes d'alcoylaminoalcools et de leurs sels pharmaceutiquement acceptables, de formule générale:

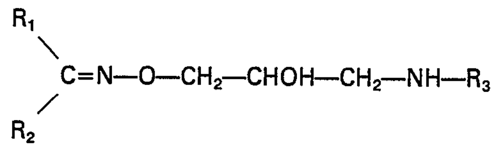 (1)

dans laquelle:

$R_1$ et $R_2$ peuvent être:

a) soit tous deux des restes cycloalcoyles $C_3$ à $C_5$;

b) soit l'un un reste alcoyle $C_1$ à $C_5$ et l'autre un reste cycloalcoyle $C_3$ à $C_5$

c) $R_1$ et $R_2$ peuvent former ensemble un cyclohexane ou un 3,3,5-triméthylcyclohexane.

$R_3$ est un radical alcoyle $C_1$ à $C_5$, linéaire ou ramifié.

2. Utilisation selon la revendication 1 où, dans la formule générale (1), $R_1$ est un radical alcoyle $C_1$ à $C_3$, $R_2$ est un radical cycloalcoyle $C_3$ à $C_5$ et $R_3$ un radical alcoyle inférieur $C_1$ à $C_5$, linéaire ou ramifié.

3. Utilisation selon la revendication 1 où, dans la formule générale (1), $R_1$ et $R_2$ sont tous deux des radicaux cycloalcoyles $C_1$ à $C_3$, $R_3$ étant un radical alcoyle inférieur $C_1$ à $C_5$, linéaire ou ramifié.

4. A titre de produits industriels nouveaux les produits de formule (1) dans laquelle:

— $R_1$ = méthyle, $R_2$ = cyclopropyle, $R_3$ = tertiobutyle

— $R_1$ = $R_2$ = cyclopropyle, $R_3$ = tertiobutyle.

5. Procédé de préparation des produits de formule (1) selon les revendications 1 à 4, caractérisé en ce que l'on fait réagir une cétone

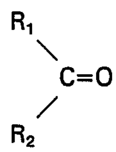

avec un sel d'hydroxylamine pour obtenir l'oxime

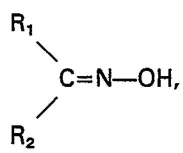

cette dernière étant mise en réaction avec une épihalohydrine pour donner un époxyde du type:

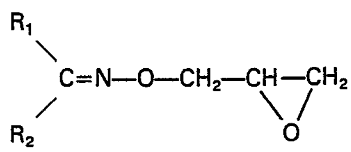

qui, en réagissant avec l'amine $R_3$—$NH_2$ conduit aux produits de formule (1).

**Patentansprüche**

1. Verwendung von Alkylaminoalkohol-Etheroximen und ihren pharmazeutisch verwendbaren Salzen der allgemeinen Formel

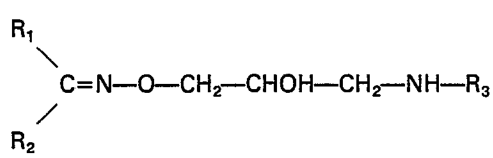 (1)

in welcher

$R_1$ und $R_2$ stehen können für

a) entweder beide für $C_3$- bis $C_5$-Cycloalkylreste oder

b) einer der Reste für einen $C_1$- bis $C_5$-Alkylrest und der andere für einen $C_3$- bis $C_5$-Cycloalkylrest oder

c) $R_1$ und $R_2$ können gemeinsam ein Cyclohexan oder ein 3,3,5-Trimethylcyclohexan bilden und

$R_3$ steht für einen linearen oder verzweigten $C_1$- bis $C_5$-Alkylrest zur Herstellung eines Medikaments zur Behandlung von Augenkrankheiten, insbesondere des Glaukoms.

2. Verwendung nach Anspruch 1, wobei in der allgemeinen Formel (1) $R_1$ für einen $C_1$- bis $C_3$-Alkylrest, $R_2$ für einen $C_3$- bis $C_5$-Cycloalkylrest und $R_3$ für einen niedrigen, linearen oder verzweigten $C_1$- bis $C_5$-Alkylrest steht.

3. Verwendung nach Anspruch 1, wobei in der allgemeinen Formel (1) $R_1$ und $R_2$ beide für $C_1$- bis $C_3$-Cycloalkylreste und $R_3$ für einen linearen oder verzweigten niedrigen $C_1$- bis $C_5$-Alkylrest steht.

4. Als neue Industrieprodukte die Produkte der Formel (1), in welcher bedeutet:

$R_1$ Methyl, $R_2$ Cyclopropyl, $R_3$ Tertiärbutyl oder

$R_1 = R_2 =$ Cyclopropyl, $R_3$ Tertiärbutyl.

5. Verfahren zur Herstellung von Produkten der Formel (1) gemäß den Ansprüchen 1 bis 4, dadurch gekennzeichnet, daß man ein Keton

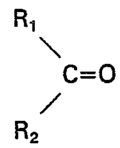

mit einem Hydroxylamin-Salz zur Gewinnung eines Oxims

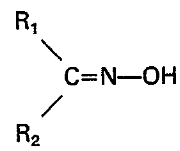

reagieren läßt, worauf dieses letztere mit einem Epihalohydrin umgesetzt wird, um ein Epoxid des Typs

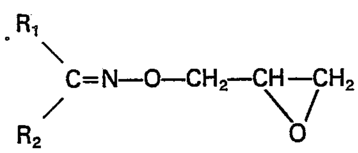

zu ergeben, welches durch Reaktion mit dem Amin $R_3$—$NH_2$ zu Produkten der Formel (1) führt.

**Claims**

1. Use for the manufacture of a medicament for the treatment of afflictions of the eye including glaucoma, of etheroximes of alkylamino alcohols and their pharmaceutically acceptable salts, of the general formula:

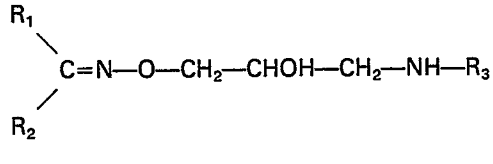

(I)

in which

$R_1$ and $R_2$:

a) may be both $C_3$—$C_5$ cycloalkyl radicals

b) one may be a $C_1$—$C_5$ alkyl radical and the other may be $C_3$—$C_5$ cycloalkyl radical

c) can jointly form a cyclohexane or a 3,3,5 trimethylcyclohexane

$R_3$ is a linear or branched $C_1$—$C_5$ alkyl radical

2. Use according the claim 1, wherein in general formula (I): R is a $C_1$—$C_3$ alkyl radical; $R_2$ is a $C_3$—$C_5$ cycloalkyl radical and $R_3$ is a linear or branched lower $C_1$—$C_5$ alkyl radical.

3. Use according the claim 1, wherein in general formula (I) $R_1$ and $R_2$ are both $C_1$—$C_3$ cycloalkyl radicals, $R_3$ being a linear or branched lower $C_1$—$C_5$ alkyl radical.

4. As new industrial compounds, the products of the general formula (I) in which:

— $R_1$ is methyl, $R_2$ is cyclopropyl, $R_3$ is tertiobutyl

— $R_1 = R_2 =$ cyclopropyl; $R_3 =$ tertiobutyl

5. A method of preparation of the products according the formula (I) according the claims 1 to 4, wherein a ketone

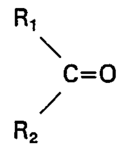

is reacted with a salt of hydroxylamine to obtain the oxime of formula:

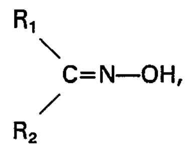

this latter being caused to react with an epihalohydrin to yield an epoxyde of the type:

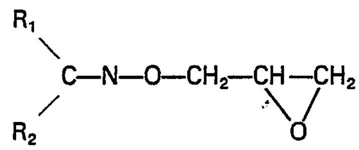

which, on the action with the amine $R_3—NH_2$ leads to products of formula (I).